# EUROPÄISCHE PATENTSCHRIFT

(11) **EP 2 252 216 B1**
(45) Veröffentlichungstag und Bekanntmachung des Hinweises auf die Patenterteilung: **07.09.2016**
(21) Anmeldenummer: 09704135.4
(22) Anmeldetag: 20.01.2009
(51) Int. Cl.: A61B 6/03, H01J 35/30, H01J 35/16

(54) **ANORDNUNG ZUR DREIDIMENSIONALEN ELEKTRONENSTRAHLTOMOGRAPHIE**
ARRANGEMENT FOR THREE-DIMENSIONAL ELECTRON BEAM TOMOGRAPHY
AGENCEMENT POUR TOMOGRAPHIE À FAISCEAU D'ÉLECTRONS EN TROIS DIMENSIONS

(30) Priorität: 23.01.2008 DE 102008005718
(43) Veröffentlichungstag der Anmeldung: 24.11.2010
(73) Patentinhaber: Helmholtz-Zentrum Dresden - Rossendorf e.V., 01328 Dresden (DE)
(72) Erfinder: HAMPEL, Uwe, 01324 Dresden (DE)
(86) Internationale Anmeldenummer: PCT/DE2009/050000
(87) Internationale Veröffentlichungsnummer: WO 2009/092372

(56) Entgegenhaltungen:
- DE-A1- 10 240 628
- DE-A1- 10 356 601
- DE-A1- 19 510 047
- US-A- 4 944 448
- US-A1- 2006 050 842
- GE WANG ET AL: "TOPICAL REVIEW; Approximate and exact cone-beam reconstruction with standard and non-standard spiral scanning" PHYSICS IN MEDICINE AND BIOLOGY, TAYLOR AND FRANCIS LTD. LONDON, GB, Bd. 52, Nr. 6, 21. März 2007 (2007-03-21), Seiten R1-R13, XP020113255 ISSN: 0031-9155

## Beschreibung

Die Erfindung betrifft eine Anordnung zur dreidimensionalen Röntgen-Computertomographie (Röntgen-CT) mit einem Elektronenstrahl.

Die Elektronenstrahl-Röntgentomographie wird seit einigen Jahren in der medizinischen Diagnostik, insbesondere zur Bildgebung des schlagenden Herzens, eingesetzt. Dabei wird ein in einer Vakuumkammer geführter Elektronenstrahl mittels eines elektromagnetischen Ablenksystems über ein teilkreisförmiges Metalltarget geführt, womit ein schnell beweglicher Röntgenbrennfleck erzeugt wird. Ein mit leichtem axialem Versatz zum Target angeordneter kreis- oder teilkreisförmiger Röntgendetektor erfasst die durch das Untersuchungsobjekt transmittierte Röntgenstrahlung. Aus den Messdaten kann dann durch Anwendung tomographischer Bildrekonstruktionsverfahren die Materialverteilung in der durchstrahlten Schnittebene berechnet werden.

Ebenfalls bekannt und seit einigen Jahren im medizinischen Einsatz sind Röntgen-Computertomographie-Scanner, die nach dem Prinzip der Spiral-CT arbeiten. Dabei wird das Objektvolumen dreidimensional gescannt, indem das Untersuchungsobjekt (zum Beispiel ein Patient auf einer Liege) langsam und kontinuierlich in axialer Richtung durch die Schnittebene bewegt wird, während das aus Röntgenstrahler und Detektor bestehende Aufnahmesystem schnell um das Untersuchungsobjekt gedreht wird. Im Objektkoordinatensystem ergibt sich daraus ein helikaler Verlauf der Röntgenbrennfleckbahn um das Objekt, woher für das Aufnahmeverfahren der Name "Spiral-Computertomographie (Spiral-CT)" rührt.

Der besondere Vorteil der Elektronenstrahl-Computertomographie-Anordnung liegt in der hohen erreichbaren Bildrate, die durch die schnelle Ablenkbarkeit des trägheitsfreien Elektronenstrahls mit Hilfe magnetischer Wechselfelder gegeben ist. Im Prinzip lassen sich aber mit dieser Anordnung nur Bilder einer Durchstrahlungsebene erzeugen. Ein Übergang zu einer Mehrschicht- oder Spiral-CT ist nur durch axiale Bewegung des Untersuchungsobjektes möglich. Allerdings ist dann wieder durch die Trägheit des Untersuchungsobjektes die Aufnahmerate für dreidimensionale Daten eingeschränkt. Eine schnelle dreidimensionale Bildgebung ist so nicht möglich. Die in den Schriften
US 4352021 A --.
und
US 5504791 A --.
beschriebenen Elektronenstrahl-Computertomographie-Anordnungen erlauben es immerhin, den Elektronenstrahl über verschiedene in axialer Richtung liegende Targetsegmente zu führen. Damit kann sehr schnell die Schnittebene gewechselt werden, womit quasi-simultane Aufnahmen in verschiedenen Schnittebenen möglich sind. Da hier aber der Schnittebenenabstand konstruktionsbedingt bei einigen Millimetern bis Zentimetern liegt, ist keine dreidimensionale Bildgebung mit hoher axialer Auflösung, zum Beispiel kleiner als im Einmillimeterbereich, möglich. Zudem ist der Röntgendetektor bei diesen Anordnungen mit einem axialen Versatz zum Target angeordnet. Dies bedeutet, dass die abtastenden Röntgenstrahlen nicht sämtlich in einer Schnittebene liegen, so dass daraus Abbildungsfehler resultieren, die die Diagnostik erschweren.

Dokument US 2006/050842 A1 offenbart eine Anordnung zur dreidimensionalen Elektronenstrahltomographie, mit einer Elektronenkanone Erzeugung, Fokussierung and Ablenkung eines Elektronenstrahls innerhalb einer Vakuumkammer, derart, dass der Elektronenstrahl in einer Elektronenstrahlrichtung verlaufend aus der Elektronenkanone austritt, Mitteln zur Fokussierung und Ablenkung des Elektronenstrahls innerhalb der Vakuumkammer, einem innerhalb der Vakuumkammer angeordneten Target zur Bremsung des Elektronenstrahls und zur Erzeugung von Röntgenstrahlung, welches wiederum aus einem Targetgrundkörper besteht, einem innerhalb der Vakuumkammer angeordnete Röntgendetektor, der in mehrere Segmente unterteilt ist, einer Aufnahmeöffnung für das Untersuchungsobjekt, einem innerhalb der Vakuumkammer angeordneten Eleketronenstrahblende, die Segmente des Röntgendetektors das Target in gewissen axialen Abständen zueinander außen umschließen, und der Elektronenstrahl mittels des elektronenoptischen Systems in eine Spiralbahn gezwungen wird und so auf der Target eine räumlich spiralförmig Brennfleckbahn erzeugt.

Aufgabe der vorliegenden Erfindung ist es, eine Anordnung zur Röntgen-Computertomographie anzugeben, die eine kontinuierliche dreidimensionale Abbildung des Untersuchungsobjektes bzw. eines Teilvolumens des Untersuchungsobjektes bei hoher zeitlicher und räumlicher Auflösung gestattet, wobei mit hoher räumlicher Auflösung sowohl eine hohe Auflösung innerhalb der Schnittebene als auch in axialer Richtung definiert ist.

Zu Lösung der Aufgabe wird eine Anordnung zur Elektronenstrahltomographie mit feststehendem und speziell geformten durchstrahlbaren Target sowie einem feststehenden Röntgendetektor angegeben. Die Aufgabe wird durch die Merkmale des Anspruchs 1 gelöst. Ausgestaltungen der Erfindung sind in den Unteransprüchen ausgeführt.

Der besondere Vorteil der erfundenen Anordnung zur Röntgen-Computertomographie besteht darin, dass mit ihr die zeitlich und räumlich kontinuierliche Abbildung der Schwächungskoeffizientenverteilung und damit hauptsächlich der Dichteverteilung in einem Objekt bei hoher Bildrate möglich ist. Damit ist die erfundene Anordnung für viele diagnostische Fragestellungen geeignet, bei denen dynamische Vorgänge untersucht werden sollen. Im biologisch-medizinischen Bereich sind dies beispielsweise Untersuchungen zur Durchblutungsdynamik und Bewegungsabläufen an Menschen und Tieren oder im technischen Bereich zum Beispiel Fragestellungen zu dynamischem Materialverhalten bei Belastung oder zu Strömungsvorgängen in Gefäßen.

Die Erfindung wird nachfolgend anhand eines Ausführungsbeispiels näher erläutert.

In den zugehörigen Abbildungen zeigen - am Beispiel eines Kleintier-CT-Scanners:

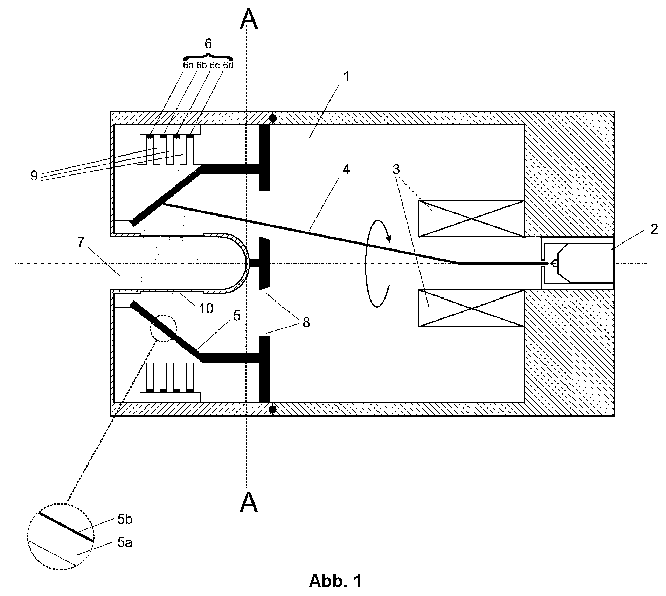

Abb. 1 einen axialen Schnitt durch die Gesamtanordnung und D

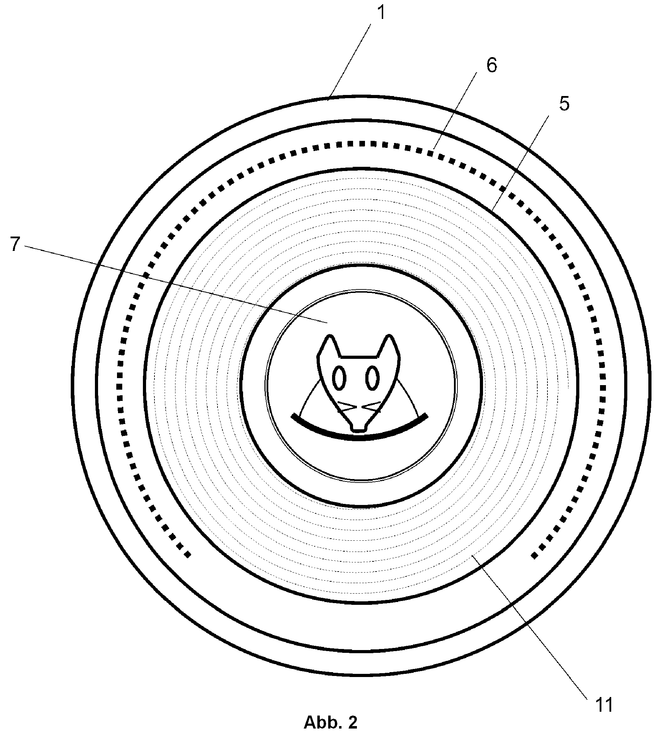

Abb. 2 eine Ansicht der Anordnung entlang des Schnittes A-A. D

Die Anordnung besteht mindestens aus folgenden Komponenten:
- einer Vakuumkammer 1 die das Elektronenstrahlsystem umschließt, D
- einer Elektronenkanone **2,** D
- einem elektronenoptischen System **3** zur Fokussierung und zweidimensionalen Ablenkung des aus der Elektronenkanone **2** austretenden Elektronenstrahls **4,** D
- einem Target **5** zur Röntgenstrahlerzeugung, D
- einem Röntgendetektor **6,** D
- Elektronenstrahlblenden **8,** D
- einem Detektorkollimator **9** zur Unterdrückung von Streustrahlung, D
- sowie optional weiteren hier nicht explizit aufgeführte, aber dem Fachmann geläufige Komponenten, wie Sensoren zur Messung und Überwachung, wichtiger Betriebsparameter (Targettemperatur, Vakuumgüte, Elektronenstrahlparameter), Elektronikkomponenten zur Messwerterfassung, Hoch- und Hilfsspannungserzeugung und Strahlführung, Komponenten zur Strahlaufhärtung, Komponenten zur Abschirmung, Liege oder Halterungen für das Untersuchungsobjekt, Kühlvorrichtung für das Target. D

Vorteilhaft ist, wenn die Aufnahmeöffnung für das Untersuchungsobjekt **7,** bei der im Innenbereich die Wandung der Vakuumkammer **1** zu einem Röntgenstrahlaustrittsfenster **10** ausgedünnt ist.

Das Target **5** hat die Form eines sich in Elektronenstrahlrichtung hin verjüngenden Zylinderrings. Das Target **5** besteht aus einem festen Targetgrundkörper **5a,** der wiederum vorteilhafterweise aus einem Material niedriger Dichte und hohem Wärmeleit- und -speichervermögen (zum Beispiel verdichtetes Graphit) besteht, sowie einer auf der Innenseite des Targetgrundkörpers **5a** aufgebrachten dünnen Wandlungsschicht **5b** aus einem Material hoher Kernladungszahl und Dichte (zum Beispiel Wolfram).

Der aus der Elektronenkanone **2** austretende Elektronenstrahl **4** wird durch das elektronenoptische System **3** auf die Innenseite des Targets **5** fokussiert. Mittels der Ablenkspulen des elektronenoptischen Systems **2** wird der Strahl dabei auf eine spiralförmige Bahn gezwungen, so dass auf der Innenseite des Targets **5** eine spiralförmige Brennfleckbahn **11** erzeugt wird. Alternativ ist es möglich, den Elektronenstrahl **4** nacheinander auf Kreisbahnen verschiedener Durchmesser durch sprunghafte Änderung des polaren Strahlablenkwinkels bei kontinuierlicher Kreisbewegung des Strahls zu fahren.

Der Röntgendetektor **6** besteht aus mehreren teilkreisförmigen Segmenten (6a, 6b, ...), die wiederum lückenlos aneinandergereihte Einzeldetektoren umfassen. Der Röntgendetektor **6** ist mit einer im Bild nicht dargestellten Spezialelektronik verbunden, die die Signale der Einzeldetektoren des Röntgendetektors **6** sehr schnell erfassen und speichern kann. Der Winkelbereich der Teilkreissegmente **6a, 6b,** ... sowie die Anzahl der Teilkreissegmente **6a, 6b,** ... wird anhand anerkannter Regeln und Gesetzmäßigkeiten der computertomographischen Bildrekonstruktion bestimmt und sind dem Fachmann als bekannt vorausgesetzt. So ergibt sich der erforderliche Winkel der Teilkreissegmente aus 180° plus dem Winkel des Strahlenfächers der Anordnung. Die Anzahl der Segmente ergibt sich aus der geforderten Bildgüte. Da es sich bei diesem Abbildungsverfahren um eine Kegelstrahl-CT handelt, ergeben sich aus der dadurch bedingten Unterabtastung des dreidimensionalen Projektionsraumes gewisse Bildunschärfen. Diese nehmen mit steigender Anzahl von Teilkreissegmenten pro axialer Einheitslänge ab.

Ein weiteres Merkmal der Anordnung sind bestimmte Hilfselemente. Zu diesen zählen Elektronenstrahlblenden **8** und Detektorkollimator **9.** Zusätzlich kann die Anordnung weitere Hilfselemente umfassen, die dem Fachmann geläufig sind und keine wesentlichen Komponenten im Sinne der Erfindung darstellen. Die Elektronenstrahlblenden **8** stellen eine Apertur für den Elektronenstrahl **4** dar, die verhindert, dass andere Elemente der Anordnung außer dem Target **5** vom Elektronenstrahl **4** getroffen werden können. Diese Elektronenstrahlblenden **8** können gleichzeitig für das Strahlmonitoring, also zum Beispiel zur Überwachung der Strahlstromkonstanz verwendet werden, indem sie zum Erdpotential isoliert montiert werden und Ableitströme über Widerstände gemessen werden, wenn der Elektronenstrahl **4** absichtlich über diese Elektronenstrahlblenden **8** geführt wird.

Der Detektorkollimator **9** begrenzt das axiale Sichtfeld der Detektorsegmente **6a, 6b,** ... auf fächerförmige Bereiche und ist axialsymmetrisch aufgebaut. Seine Aufgabe ist die Unterdrückung von Streustrahlung aus Konstruktionselementen der Anordnung.

Bezugszeichenliste
1 Vakuumkammer D
2 Elektronenkanone D
3 Elektronenoptisches System D
4 Elektronenstrahl D
5 Target D
5a Targetgrundkörper D
5b Wandlungsschicht D
6 Röntgendetektor D
6a ... 6d Teilkreissegmente des Röntgendetektors D
7 Aufnahmeöffnung für das Untersuchungsobjekt D
8 Elektronenstrahlblenden D
9 Detektorkollimator D
10 Röntgenstrahlaustrittsfenster D
11 Brennfleckbahn D

## Patentansprüche

1. Anordnung zur dreidimensionalen Elektronenstrahltomographie, mit:
- einer Elektronenkanone (2) zur Erzeugung eines Elektronenstrahls (4) innerhalb einer Vakuumkammer (1) derart, dass der Elektronenstrahl in einer Elektronenstrahlrichtung verlaufend aus der Elektronenkanone austritt,
- einem elektronenoptischen System (3) zur Fokussierung und Ablenkung des Elektronenstrahls (4) innerhalb der Vakuumkammer (1),
- einem innerhalb der Vakuumkammer (1) angeordneten Target (5) zur Bremsung des Elektronenstrahls (4) und zur Erzeugung von Röntgenstrahlung, welches wiederum aus einem Targetgrundkörper (5a) und einer auf der Innenseite des Targetgrundkörpers aufgebrachten Wandlungsschicht (5b) besteht, wobei das Target (5) derart ausgebildet ist, dass es von der Röntgenstrahlung durchstrahlbar ist,
- einem innerhalb der Vakuumkammer (1) angeordneten Röntgendetektor (6), der in mehrere Teilkreissegmente (6a, 6b, 6c, 6d) unterteilt ist,
- einer Aufnahmeöffnung für das Untersuchungsobjekt (7),
- innerhalb der Vakuumkammer (1) angeordneten Elektronenstrahlblenden (8) und
- einem innerhalb der Vakuumkammer (1) angeordneten Detektorkollimator (9), wobei
- das Target (5) die Aufnahmeöffnung für das Untersuchungsobjekt (7) umschließt,
- das Target (5) in Form eines sich in die bei Austritt aus der Elektronenkanone (2) vorliegende Elektronenstrahlrichtung hin verjüngenden Zylinderrings gestaltet ist,
- die Teilkreissegmente (6a, 6b, 6c, 6d) des Röntgendetektors (6) das Target (5) in gewissen axialen Abständen zueinander außen umschließen,
- der Elektronenstrahl (4) mittels des elektronenoptischen Systems (3) in eine Spiralbahn gezwungen wird und so auf der Innenseite des Targets (5) eine räumlich spiralförmige Röntgenbrennfleckbahn (11) erzeugt oder mittels des elektronenoptischen Systems (3) nacheinander in Kreisbahnen verschiedener Durchmesser gezwungen wird und so auf der Innenseite des Targets (5) eine räumliche Röntgenbrennfleckbahn (11) erzeugt wird, die aus einer Vielzahl von Kreisen verschiedener Durchmesser besteht.

2. Anordnung nach Anspruch 1, **dadurch gekennzeichnet, dass** der Detektorkollimator (9) das axiale Sichtfeld der Detektorsegmente (6a, 6b, 6c, 6d) auf fächerförmige Bereiche begrenzt und axialsymmetrisch aufgebaut ist.

3. Anordnung nach einem der vorhergehenden Ansprüche, **dadurch gekennzeichnet, dass** Elektronenstrahlblenden (8) derart in den Elektronenstrahlverlauf angeordnet sind, so dass nur das Auftreffen des Elektronenstrahls auf das Target gewährleistet wird.

4. Anordnung nach einem der vorhergehenden Ansprüche, **dadurch gekennzeichnet, dass** das Wandmaterial der Vakuumkammer (1) im Bereich der Aufnahmeöffnung für das Untersuchungsobjekt (7) zu einem Röntgenstrahlaustrittsfenster (10) ausgedünnt ist.

## Claims

1. Arrangement for three-dimensional electron beam tomography, comprising:
- an electron gun (2) for generating an electron beam (4) within a vacuum chamber (1) such that the electron beam exits from the electron gun in an electron beam direction,
- an electron-optical system (3) for focusing and deflecting the electron beam (4) within the vacuum chamber (1),
- a target (5) arranged within the vacuum chamber (1) for decelerating the electron beam (4) and for generating X-ray radiation, which target in turn comprises a target base body (5a) and a conversion layer (5b) applied to the inner side of the target base body, wherein the target (5) is designed in such a way that the X-ray radiation can radiate through it,
- an X-ray detector (6) subdivided into a plurality of circular-arc segments (6a, 6b, 6c, 6d) and arranged within the vacuum chamber (1),
- a receiving opening for the examination object (7),
- electron beam stops (8) arranged within the vacuum chamber (1) and
- a detector collimator (9) arranged within the vacuum chamber (1), wherein
- the target (5) surrounds the receiving opening for the examination object (7),
- the target (5) is designed as a cylindrical ring tapering in the electron beam direction present when exiting from the electron gun (2),
- the circular-arc segments (6a, 6b, 6c, 6d) of the X-ray detector (6) surround the target (5) on the outside with certain axial distances from one another,
- the electron beam (4) is forced into a spiral path by means of the electron-optical system (3) and thereby generates a spatially spiral-shaped focal-spot path (11) on the inside of the target (5) or the electron beam is successively forced into circular paths with different diameters by means of the electron-optical system (3) and thereby generates a spatial focal-spot path (11) that consists of a multiplicity of circles with different diameters on the inside of the target (5).

2. Arrangement according to Claim 1, **characterized in that** the detector collimator (9) limits the axial field of view of the detector segments (6a, 6b, 6c, 6d) to fan-shaped regions and has an axially symmetric design.

3. Arrangement according to one of the preceding claims, **characterized in that** electron beam stops (8) are arranged in the electron beam path in such a way that only the incidence of the electron beam on the target is ensured.

4. Arrangement according to one of the preceding claims, **characterized in that** the wall material of the vacuum chamber (1) is thinned out to form an X-ray beam output window (10) in the region of the receiving opening for the examination object (7).

## Revendications

1. Agencement pour la tomographie à faisceau électronique tridimensionnelle, avec :
- un canon électronique (2) destiné à générer un faisceau électronique (4) à l'intérieur d'une chambre à vide (1) de telle sorte que le faisceau électronique sorte du canon électronique en s'écoulant dans une direction de faisceau électronique,
- un système optoélectronique (3) pour focaliser et dévier le faisceau électronique (4) à l'intérieur de la chambre à vide (1),
- une cible (5) placée à l'intérieur de la chambre à vide (1) pour freiner le faisceau électronique (4) et pour générer un rayonnement X, laquelle quant à elle est constituée d'un corps de base (5a) de cible et d'une couche de conversion (5b) appliquée sur la face intérieure du corps de base de la cible, la cible (5) étant conçue de sorte à pouvoir être traversée par le rayonnement X,
- un détecteur de rayons X (6) placé à l'intérieur de la chambre à vide (1), lequel est divisé en plusieurs segments de cercle gradué (6a, 6b, 6c, 6d),
- un orifice de logement pour l'objet (7) à examiner,
- des diaphragmes (8) pour faisceaux électroniques placés à l'intérieur de la chambre à vide (1),
et
- un collimateur de détecteur (9) placé à l'intérieur de la chambre à vide (1),
- la cible (5) entourant l'orifice de logement pour l'objet (7) à examiner,
- la cible (5) étant conçue sous la forme d'un anneau cylindrique se rétrécissant dans la direction du faisceau électronique qui est présent à la sortie du canon électronique (2),
- les segments de cercle gradué (6a, 6b, 6c, 6d) du détecteur de rayons X (6) entourant la cible (5) à l'extérieur, avec certains écarts axiaux mutuels,
- au moyen du système optoélectronique (3), le faisceau électronique (4) étant forcé dans une trajectoire hélicoïdale et il est généré ainsi sur la face intérieure de la cible (5) un trajet focal (11) télescopique de rayon X hélicoïdal ou au moyen du système optoélectronique (3) étant forcé successivement dans des orbites de différents diamètres et il est généré ainsi sur la face intérieure de la cible (5) un trajet focal (11) télescopique de rayon X qui est constitué d'une pluralité de cercles de différents diamètres.

2. Agencement selon la revendication 1, **caractérisé en ce que** le collimateur de détecteur (9) limite le champ visuel axial des segments de détecteur (6a, 6b, 6b, 6d) à des zones en éventail et est conçu en étant axisymétrique.

3. Agencement selon l'une quelconque des revendications précédentes, **caractérisé en ce que** des diaphragmes (8) pour faisceaux électroniques sont placés dans le trajet du faisceau électronique de sorte que seule l'incidence du faisceau électronique sur la cible soit assurée.

4. Agencement selon l'une quelconque des revendications précédentes, **caractérisé en ce que** dans la zone de l'orifice de logement pour l'objet (7) à examiner, la matière de la paroi de la chambre à vide (1) est amincie en une fenêtre de sortie (10) de rayons X.
